# EUROPÄISCHE PATENTSCHRIFT

(11) **EP 2 574 609 B1**
(45) Veröffentlichungstag und Bekanntmachung des Hinweises auf die Patenterteilung: **30.03.2016**
(21) Anmeldenummer: 12184670.3
(22) Anmeldetag: 17.09.2012
(51) Int. Cl.: C07D 307/50

(54) **Verfahren zur Herstellung von Furanverbindungen aus nachwachsenden Rohstoffen**
Method for manufacturing furan compounds from renewable resources
Procédé de fabrication de liaisons de furane à partir de matières premières renouvelables

(30) Priorität: 30.09.2011 EP 11007974
(43) Veröffentlichungstag der Anmeldung: 03.04.2013
(73) Patentinhaber: Fraunhofer-Gesellschaft zur Förderung der angewandten Forschung e.V., 80686 München (DE)
(72) Erfinder: Forstner, Jochen, 75397 Simmozheim (DE); Schweppe, Rainer, 76228 Karlsruhe (DE); Unkelbach, Gerd, 76768 Berg (DE); Flick, Klemens, Dr., 67165 Waldsee (DE)
(74) Vertreter: Maiwald Patentanwalts GmbH

(56) Entgegenhaltungen:
- WO-A1-2010/030196
- WO-A1-2011/063500
- WO-A2-2010/075437
- CN-A- 1 974 382
- CN-A- 101 787 006
- DE-A1- 19 619 075
- DE-B- 1 191 734
- US-B2- 8 053 615
- CHAREONLIMKUN A ET AL: "Reactions of C5 and C6-sugars, cellulose, and lignocellulose under hot compressed water (HCW) in the presence of heterogeneous acid catalysts", FUEL, IPC SCIENCE AND TECHNOLOGY PRESS, GUILDFORD, GB, Bd. 89, Nr. 10, 1. Oktober 2010 (2010-10-01), Seiten 2873-2880, XP027136755, ISSN: 0016-2361 [gefunden am 2010-03-17]
- MCNEFF C V ET AL: "Continuous production of 5-hydroxymethylfurfural from simple and complex carbohydrates", APPLIED CATALYSIS A: GENERAL, ELSEVIER SCIENCE, AMSTERDAM, NL, Bd. 384, Nr. 1-2, 20. August 2010 (2010-08-20), Seiten 65-69, XP027189338, ISSN: 0926-860X [gefunden am 2010-06-11]

## Beschreibung

Die vorliegende Erfindung betrifft die Herstellung der Furanverbindungen Furfural und 5-Hydroxymethylfurfural aus nachwachsenden Rohstoffen. In diesem Zusammenhang beschreibt die Erfindung ein einfaches und kostengünstiges Verfahren zur Herstellung dieser Furanverbindungen, in Gegenwart eines bestimmten heterogenen Katalysators aus einer wässrigen Lösung. Außerdem beschreibt die vorliegende Beschreibung einen heterogenen Katalysator, sowie ein Verfahren zu dessen Herstellung.

### Hintergrund der Erfindung

Verknappung und steigende Preise bei fossilen Rohstoffen haben dazu geführt, dass ein verstärktes Interesse an nachhaltigen Synthesestrategien besteht. Dabei kommt der Synthese von chemischen Grundstoffen, welche typischerweise durch petrochemische Raffination gewonnen werden, ein ganz besonderes Interesse zu. Es wurde ein erheblicher Aufwand betrieben, alternative Synthesestrategien zu entwickeln, um die Bedürfnisse der chemischen Industrie auch in Zukunft decken zu können. Ein besonders erfolgreiches Konzept in diesem Zusammenhang ist die Bioraffination. Die "Bioraffination" bezeichnet ein technisches Verfahren zur Reinigung, Veredlung, Trennung und/oder Konzentration von einzelnen Rohstoffen aus einer Biomasse, wobei unter "Biomasse" nachwachsende Rohstoffe verstanden werden. Die aus Biomasse entstandenen fossilen Energieträger hingegen, werden den nachwachsenden Rohstoffen nicht zugeordnet. Biomasse enthält im Allgemeinen etwa 35-50% Cellulose, 25-30% Hemicellulose und 25-30% Lignin. Hemicellulose ist dabei als ein Sammelbegriff für die in pflanzlicher Biomasse vorkommenden Gemische aus Polysacchariden zu verstehen. Im Rahmen der Bioraffination können Saccharide wie Glucose, Xylose, und Fructose erhalten werden.

Die vorliegende Erfindung betrifft die Herstellung der Furanverbindungen Furfural oder 5-Hydroxymethylfurfural aus Sacchariden. In diesem Zusammenhang beschreibt die Erfindung ein einfaches und kostengünstiges Verfahren zur Herstellung dieser Furanverbindungen in Gegenwart eines bestimmten heterogenen Katalysators. Furanverbindungen sind von besonderem Interesse, da sie wertvolle Intermediate bei der Herstellung von Polymeren, Lösungsmitteln, pharmazeutischen Erzeugnissen, Feinchemikalien, Treibstoffen, Düngemitteln und Herbiziden darstellen. Es werden zumeist hohe Anforderungen an die Reinheit der benötigten Furanverbindungen gestellt.

Die Herstellung von Furanverbindungen aus nachwachsenden Rohstoffen ist bekannt und bereits technisch realisiert. Die Herstellung der Furanverbindungen, insbesondere von Furfural und 5-Hydroxymethylfurfural gestaltet sich jedoch schwierig, da Folgereaktionen zu diversen Abbauprodukten führen. Bei den bekannten Verfahren zur Herstellung der Furanverbindungen kommen homogene Katalysatoren zum Einsatz. Unter "homogene Katalysatoren" sind solche Katalysatorsysteme zu verstehen, bei denen Katalysator und Edukt in derselben Phase vorliegen. Bei den bekannten Verfahren werden als Katalysatoren üblicherweise anorganische Säuren eingesetzt. Beispielsweise wird in "Reactions of d-fructose in water at temperatures up to 400°C and pressures up to 100MPa, Aida T.M. et al., Journal of Supercritical Fluids 2007, 42(1), 110-119, die Herstellung von Furanverbindungen in Gegenwart von homogenen Säuren, insbesondere anorganischen homogenen Säuren wie Schwefelsäure, Phosphorsäure oder Salzsäure beschrieben.

CN 101787006 offenbart ein Verfahren zur Gewinnung von Furanen aus Biomasse.

Durch den Einsatz des homogenen Katalysators ist eine aufwändige und kostenintensive Aufarbeitung nötig. Die Säuren müssen beispielsweise zunächst neutralisiert werden, um die Reaktion zu stoppen und so die weitere Aufarbeitung erst zu ermöglichen. Außerdem werden in den bekannten Verfahren organische Lösemittel, ionische Flüssigkeiten oder überkritische Fluide eingesetzt, was eine Aufarbeitung zusätzlich erschwert und die Produktionskosten erhöht. Der Einsatz von organischen Lösemitteln oder anderen Hilfsstoffen wirkt sich dabei insbesondere negativ auf die Reinheit der erhaltenen Furanverbindungen aus.

Es besteht deshalb ein Bedarf an neuen Katalysatoren, Katalysatorsystemen oder Reaktionssystemen, mit denen die Herstellung von Furanverbindungen verbessert werden kann. Ein besonderes Interesse liegt dabei in einer Optimierung des Herstellungsverfahrens, um so einen geringeren Produktionspreis zu erzielen.

### Kurzdarstellung der Erfindung

Die der Erfindung zugrundeliegende Aufgabe ist die Bereitstellung eines verbesserten Verfahrens zur Herstellung der Furanverbindungen Furfural oder 5-Hydroxymethylfurfural, wobei in dem Verfahren Edukte eingesetzt werden, welche aus nachwachsenden Rohstoffen isoliert werden können. Unter "Edukt" ist eine Ausgangsverbindung zu verstehen, mit der eine chemische Reaktion durchgeführt wird, um daraus ein Produkt zu erhalten.

Diese Aufgabe wird mit den Merkmalen der unabhängigen Ansprüche gelöst. Vorteilhafte Ausgestaltungen der Erfindung sind in den Unteransprüchen angegeben. Die Erfindung betrifft ein einfaches und kostengünstiges Verfahren zur Herstellung der Furanverbindungen Furfural oder 5-Hydroxymethylfurfural, in Gegenwart von zumindest einem heterogenen Katalysator. Die Herstellung der Furanverbindungen erfolgt aus wässriger Lösung. Unter "heterogener Katalysator" sind solche Katalysatorsysteme zu verstehen, bei denen Katalysator und Edukt in unterschiedlichen Phasen vorliegen. Der Begriff *"heterogener Katalysator"* umfasst dabei sowohl Katalysatorsysteme, welche ausschließlich aus der katalytisch aktiven Verbindung bestehen als auch Katalysatorsysteme, welche neben der katalytisch aktiven Verbindung weitere Bestandteile enthalten, wie bei geträgerten Katalysatoren der Fall.

Das erfindungsgemäße Verfahren wird zur Herstellung von Furfural und 5-Hydroxymethylfurfural eingesetzt.

Das erfindungsgemäße Verfahren umfasst das Herstellen einer Eduktlösung, vorzugsweise einer wässrigen Eduktlösung. Diese Eduktlösung enthält mindestens ein Saccharid, insbesondere Xylose und/oder Fructose. In anderen Worten umfasst das erfindungsgemäße Verfahren das Bereitstellen von mindestens einem Saccharid, vorzugsweise Xylose und/oder Fructose, insbesondere in Form einer wässrigen Lösung. Anschließend wird ein heterogener Katalysator zu der Eduktlösung gegeben und das Reaktionsgemisch für 5 bis 240 Minuten auf 140 bis 250°C, insbesondere für 10 bis 60 Minuten auf 150 bis 200°C, erhitzt. Dieser Bereich ist von besonderer Bedeutung, da bei kürzerer Reaktionsdauer und/oder tieferen Temperaturen keine zufriedenstellenden Umsätze und Ausbeuten erzielt werden und bei einer längeren Reaktionsdauer und/oder höheren Temperaturen eine merkliche Zersetzung der Reaktionsprodukte auftritt. Daraufhin wird der heterogene Katalysator mittels Filtration oder anderen Methoden zur Trennung heterogener Gemische aus dem Reaktionsgemisch entfernt und die Produktlösung, beinhaltend Furfural oder 5-Hydroxymethylfurfural, abgetrennt.

Die Beschreibung beschreibt außerdem einen heterogenen Katalysator und dessen Herstellung, sowie die Verwendung des Katalysators und einer Katalysatorgruppe in dem erfindungsgemäßen Verfahren zur Dehydratisierung von Alkoholen oder Polyalkoholen, insbesondere zur Herstellung der Furanverbindungen Furfural oder 5-Hydroxymethylfurfural. Der heterogene Katalysator enthält ein Metallphosphat und/oder Übergangsmetallphosphat, ausgewählt aus FePO₄, BPO₄, AlPO₄, Ti₃(PO₄)₄ und/oder GaPO₄ oder Mischungen davon. In einer Ausführungsform hat der heterogene Katalysator eine spezifische Oberfläche in einem Bereich von 75 bis 250 m²/g, vorzugsweise von 100 bis 200 m²/g, insbesondere von 130 bis 180 m²/g.

Es sind verschiedene Verfahren für die Herstellung des heterogenen Katalysators denkbar. Zumeist erfolgt die Herstellung über eine entsprechende Vorläuferverbindung. In einer Ausführungsform wird die Vorläuferverbindung zunächst zerkleinert und anschließend in einem geeigneten Reaktionsmedium erhitzt. Die Vorläuferverbindung wird dabei vorzugsweise vollständig gelöst und der Katalysator wird nach beendeter Reaktion ausgefällt, wodurch der heterogene Katalysator erhalten wird. In einer weiteren Ausführungsform wird der Katalysator in Gegenwart eines geeigneten Trägermaterials ausgefällt. Unter "Trägermaterial" sind solche Materialien zu verstehen, welche mit dem Katalysator vermengt oder beschichtet werden, so dass ein festes Katalysatorsystem erhalten wird. Vorzugsweise handelt es sich bei dem Trägermaterial um ein poröses Material.

Der heterogene Katalysator wird anschließend kalziniert. Unter "Kalzinierung" ist das Erhitzen eines Materials zu verstehen mit dem Ziel, dieses zu entwässern. Die Kalzinierung wird in einem Bereich von 200 bis 1200°C durchgeführt und erfolgt über einem Zeitraum 1 bis 10 Stunden.

In einer bevorzugten Ausführungsform erfolgt das erfindungsgemäße Verfahren zur Herstellung einer Furanverbindung in wässriger Lösung in Gegenwart eines Katalysators, dessen Herstellung einen Kalzinierungsschritt bei 200 bis 1000°C, vorzugsweise 400 bis 1000°C, insbesondere bei 400 bis 600°C umfasst.

In einer weiteren bevorzugten Ausführungsform wird der Katalysator oder das Katalysatorsystem getrocknet. Die Trocknung erfolgt in einem Temperaturbereich von 50°C bis 350°C, vorzugsweise 75°C bis 250°C, insbesondere 100°C bis 200°C und über einen Zeitraum von 1 bis 48 Stunden, vorzugsweise 1 bis 24, insbesondere 12 bis 24. In einer besonders bevorzugten Ausführungsform erfolgt die Trocknung zusätzlich zu der Klazinierung, insbesondere vor der Klazinierung.

Der heterogene Katalysator wird erfindungsgemäß zur Herstellung von Furfural und 5-Hydroxymethylfurfural verwendet, wobei die Herstellung aus wässriger Lösung erfolgt.

### Beschreibung der Figuren

Figur 1 zeigt eine schematische Darstellung eines kontinuierlich betreibbaren Synthesereaktors.
Figur 2 zeigt den Einfluss der Kalzinierungstemperatur auf die Produktausbeute und die spezifische Katalysatoroberfläche.
Figur 3 zeigt den Einfluss auf die Aktivität des Katalysators und den pH-Wert der Produktlösung bei einer wiederholten Verwendung eines Zirkonphosphat-Katalysators (Nicht Teil der Erfindung).

### Detaillierte Beschreibung der Erfindung

Die Erfindung betrifft ein einfaches und kostengünstiges Verfahren zur Herstellung von Furanverbindungen aus wässriger Lösung, in Gegenwart eines heterogenen Katalysators. Insbesondere betrifft die Erfindung ein Verfahren zur Herstellung von Furanverbindungen aus einer wässrigen Saccharidlösung. In einer bevorzugten Ausführungsform der Erfindung wird das Saccharid, das zur Bereitstellung der wässrigen Saccharidlösung eingesetzt wird, aus einem Bioraffinationsverfahren gewonnen. In einer weiteren bevorzugten Ausführungsform wird das Saccharid, direkt aus einer biogenen Quelle erhalten.

Eine Bioraffinerie ist ein komplexes und integriertes System von Prozessen und Anlagen, in welchem Biomasse in eine Vielzahl von Produkten umgewandelt wird, welches an dem Konzept einer petrochemischen Raffinerie angelehnt ist. Unter einem Bioraffinationsverfahren ist die Übertragung von Effizienz und Logik der fossil-basierten Chemischen und Stoffwandelnden Industrie zu verstehen, wobei nicht fossile Ausgangsstoffe wie Erdöl oder Erdgas, sondern Biomassen jedweder Zusammensetzung gewandelt werden.

Eine besondere Ausführungsform der Erfindung betrifft ein Verfahren zur Herstellung von Furfural aus einer wässrigen Xyloselösung, in Gegenwart eines heterogenen Katalysators. Eine weitere besondere Ausführungsform der Erfindung betrifft ein Verfahren zur Herstellung von 5-Hydroxymethylfurfural aus einer wässrigen Fructoselösung, in Gegenwart eines heterogenen Katalysators. Eine weitere besondere Ausführungsform der Erfindung betrifft ein Verfahren zur Herstellung von Furfural und/oder 5-Hydroxymethylfurfural aus einer wässrigen Xylose- und/oder Fructoselösung, in Gegenwart eines heterogenen Katalysators. Insbesondere können Xylose und Fructose Intermediate des erfindungsgemäßen Verfahrens darstellen, welche durch Isomerisierung aus den als Edukt eingesetzten Alkoholen oder Polyalkoholen entstehen, wobei Isomerisierung einen Vorgang bezeichnet, bei dem durch intramolekular ablaufende Umwandlungen einer chemischen Verbindung ein Isomer erzeugt wird. In einer besonderen Ausführungsform wird Xylose und/oder Fructose während dem erfindungsgemäßen Verfahren aus Cellulose, insbesondere Saccharose vorzugsweise Glucose erhalten.

Das erfindungsgemäße Verfahren umfasst zumindest die folgenden Schritte:

### a) Bereitstellen einer wässrigen Eduktlösung.

Die Eduktlösung enthält zumindest ein Saccharid, ausgewählt aus der Gruppe Glycerinaldehyd, Threose, Erythrose, Ribose, Arabinose, Xylose, Lynose, Allose, Altrose, Glucose, Mannose, Gulose, Idose, Galactose, Talose, Dihydroxyaceton, Erythrulose, Ribulose, Xylulose, Psicose, Fructose, Sorbose und Tagatose, insbesondere Xylose oder Fructose. Vorzugsweise wird eine Eduktlösung bereitgestellt, welche einen Saccharidgehalt 1 bis 50 Gewichtsprozent aufweist, vorzugsweise 1 bis 25 Gewichtsprozent, insbesondere 1 bis 10 Gewichtsprozent.

### b) Zugeben eines heterogenen Katalysators.

Ein heterogener Katalysator wird zu der Eduktlösung gegeben. Der heterogene Katalysator umfasst ein Metallphosphat und/oder Halbmetallphosphat und/oder Übergangsmetallphosphat, von Metallen ausgewählt aus Fe, B, Al, Ti und Ga. In einer erfindungsgemäßen Ausführungsform wird der heterogene Katalysator in einer Menge zugegeben, so dass das Gewichtsverhältnis zwischen Katalysator und Edukt in einem Bereich zwischen 1:50 und 1:2 liegt, vorzugsweise in einem Bereich zwischen 1:10 und 1:5. In einer besonderen Ausführungsform wird der Katalysator in einer Menge zugegeben, so dass das Gewichtsverhältnis zwischen Katalysator und Edukt 1:25, vorzugsweise 1:10, insbesondere 1:5 beträgt.

### c) Erhitzen der Reaktionslösung.

Das Reaktionsgemisch wird für einen Zeitraum von 1 bis 240 Minuten, vorzugsweise 1 bis 120 Minuten, insbesondere 10 bis 60 Minuten auf 100 bis 250°C, vorzugsweise 150 bis 200°C, insbesondere 160 bis 180°C erhitzt. Besonders bevorzugt wird bei der Herstellung von Furfural eine Reaktionszeit von 60 Minuten bei 160°C und bei der Herstellung von 5-Hydroxymethylfurfural eine Reaktionszeit von 30 Minuten bei 180°C. Für die Herstellung von Furfural aus Xylose, in Gegenwart von AlPO₄, eignet sich insbesondere eine Reaktionszeit von 30 Minuten bei 200°C.

### d) Abtrennen des heterogenen Katalysators.

Anschließend wird der heterogene Katalysator aus dem Reaktionsgemisch entfernt. In einer besonderen Ausführungsform wird das Reaktionsgemisch auf Raumtemperatur abgekühlt bevor der heterogene Katalysator aus dem Reaktionsgemisch entfernt wird. Es sind verschiedene Techniken bekannt, um einen heterogenen Stoff, insbesondere einen heterogenen Katalysator aus einem Reaktionsgemisch zu entfernen, welche im Rahmen der Erfindung verwirklicht werden können. Insbesondere bevorzugt sind das Abtrennen des Katalysators mittels Filtration oder Zentrifugation. Der heterogene Katalysator lässt sich leicht aus dem Reaktionsgemisch entfernen, so dass die Reaktionszeit besonders gut gesteuert werden kann und eine genaue Abstimmung der Prozessparameter möglich ist.

### e) Isolieren des Produkts

Letztlich wird das Produkt, vorzugsweise eine Furanverbindung, insbesondere Furfural oder 5-Hydroxymethlyfurfural aus der Reaktionslösung isoliert. Auch hier sind verschiedene Techniken bekannt, welche im Rahmen der Erfindung verwirklicht werden können. In einer Ausführungsform erfolgt die Isolierung von Furfural oder 5-Hydroxymethlyfurfural aus der wässrigen Reaktionslösung durch einen einfachen Extraktionsschritt mit einem geeigneten Lösungsmittel oder Lösungsmittelgemisch. In einer besonderen Ausführungsform umfasst das Lösungsmittel oder Lösungsmittelgemisch ein organisches Lösungsmittel, vorzugsweise ein Keton und/oder Alkohol, insbesondere ein aromatisches Keton und/oder einen aromatischen Alkohol.

In einer weiteren Ausführungsform erfolgt die Isolierung des Produkts, Furfural oder 5-Hydroxymethlyfurfural aus der Reaktionslösung durch Destillation und/oder Rektifikation. In einer besonders bevorzugten Ausführungsform umfasst die Isolierung des Produkts mindestens zwei Destillationsschritte, wobei in einem ersten Destillationsschritt Verbindungen mit einer geringeren Siedetemperatur als die Zielverbindung über den Kopf der Destillations- oder Rektifikationsvorrichtung entfernt werden, und in einem zweiten Destillationsschritt Verbindungen mit einer höheren Siedetemperatur als die Zielverbindung über den Sumpf der Destillations- oder Rektifikationsvorrichtung entfernt werden. Der zweite Destillationsschritt erfolgt vorzugsweise in einer zweiten Destillations- oder Rektifikationsvorrichtung.

Die Destillation und/oder Rektifikation, kann alternativ oder zusätzlich zu der Extraktion erfolgen.

In einer erfindungsgemäßen Ausführungsform wird die Furanverbindung in einem ersten Verfahrensschritt durch Extraktion mit einem geeigneten Lösungsmittel oder Lösemittelgemisch aus der Produktlösung abgetrennt. Das Lösemittel oder Lösemittelgemisch umfasst ein organisches Lösungsmittel, vorzugsweise ein Keton und/oder Alkohol, insbesondere ein aromatisches Keton und/oder einen aromatischen Alkohol. Die Furanverbindung, wird anschließend in einem zweiten Verfahrensschritt der eine Destillation und/oder Rektifikation umfasst isoliert.

In einer weiteren bevorzugten Ausführungsform wird die Furanverbindung in einem ersten Destillationsschritt über den Sumpf und anschließend in einem zweiten Destillationsschritt über den Kopf der Destillations- oder Rektifikationsvorrichtung. abgetrennt. Auf diese Weise können Furanverbindungen mit besonders hoher Reinheit erhalten werden. Der erste und der zweite Destillationsschritt können dabei in derselben oder in verschiedenen Destillations- oder Rektifikationsvorrichtungen. erfolgen.

Das erfindungsgemäße Verfahren zeichnet sich insbesondere dadurch aus, dass die Reaktionszeit gegenüber den bekannten Verfahren deutlich herabgesetzt werden konnte und gleichzeitig Umsatzraten und Ausbeuten erhöht werden konnten. Das erfindungsgemäße Verfahren kann außerdem bei verhältnismäßig hohen Temperaturen durchgeführt werden, ohne dass ein signifikanter katalytischer und/oder thermischer Abbau der Produkte, insbesondere zu Huminstoffen und organischen Säuren eintritt. Während bei den bekannten Verfahren zur Herstellung von Furanverbindungen, insbesondere Furfural oder 5-Hydroxymethlyfurfural, eine Reaktionszeit von mehreren Stunden üblich ist, liegt die Reaktionszeit des erfindungsgemäßen Verfahrens in einem Bereich von 1 bis 120 Minuten, insbesondere 1 bis 60 Minuten, weiter bevorzugt 10 bis 60 Minuten, insbesondere 30 bis 60 Minuten.

Ein weiter entscheidender Vorteil des erfindungsgemäßen Verfahrens gegenüber den bekannten Verfahren ist, dass keinerlei organische Lösemittel, ionische Flüssigkeiten, überkritische Fluide oder sonstige Hilfsmittel wie beispielsweise Phasenhilfsmittel eingesetzt werden, welche einen zusätzlichen Aufarbeitungsschritt erforderlich machen. In dem heterogen katalysierten Verfahren werden im Vergleich zu dem homogen katalysierten Verfahren Produkte mit einer höheren Reinheit erhalten.

Das erfindungsgemäße Verfahren kann sowohl im Batchbetrieb als auch im kontinuierlichen Betrieb durchgeführt werden. Der Batchbetrieb wird vorzugsweise mit einem Stahlautoklaven durchgeführt, während der kontinuierliche Betrieb vorzugsweise in einem Strömungsrohrreaktor oder einem kontinuierlich betriebenen Rührkesselreaktor durchgeführt wird. Es hat sich gezeigt, dass der Einsatz von geträgerten Katalysatoren besonders für den Einsatz in einem Strömungsrohrreaktor geeignet ist, während sich für den Einsatz in einem kontinuierlich betriebenen Rührkesselreaktor Katalysatoren in Partikelform in besonderem Maße eignen. Die Abtrennung des heterogenen Katalysators kann im Batchbetrieb durch einfache Filtration erfolgen, während im kontinuierlichen Betrieb die Rückgewinnung des Katalysators vorzugsweise durch Sintermetallfilter, inerte Schüttungen, feinmaschige Edelstahlfilter oder Querschnittsverengungen erfolgt.

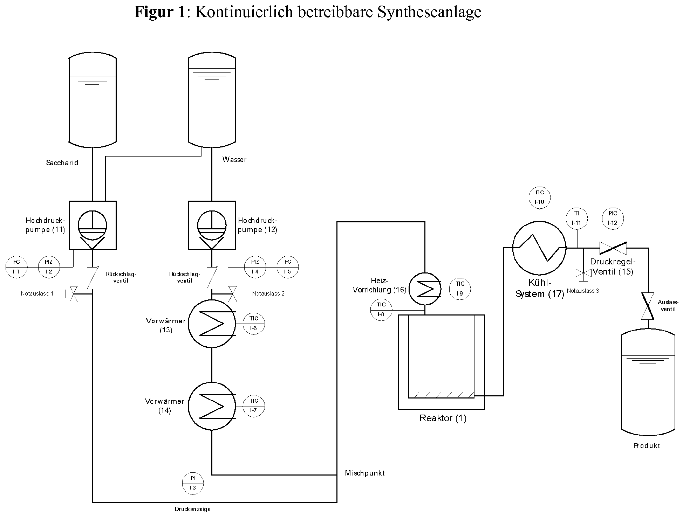

Gemäß der in Figur 1 dargestellten Ausführungsform umfasst die kontinuierlich betriebene Syntheseanlage zumindest eine Hochdruckpumpe, zumindest einen Vorwärmer zur Erwärmung des Reaktionsmediums, insbesondere Wasser, einen Reaktor, insbesondere einen beheizten Reaktor, zumindest ein Kühlsystem, insbesondere einen Flüssig-Flüssig-Wärmetauscher, und zumindest ein Druckregelventil.

Das erfindungsgemäße Verfahren umfasst dabei sowohl Einstrang- als auch Mehrstrangverfahren, wobei unter "Einstrangverfahren" ein Verfahren mit einer Hochdruckpumpe zu verstehen ist, während unter Mehrstrangverfahren ein Verfahren mit mehr als einer Hochdruckpumpe zu verstehen ist, insbesondere ein Verfahren mit mehr als einer Hochdruckpumpe und zumindest einer Mischstelle.

Außerdem beschreibt die vorliegende Beschreibung einen heterogenen Katalysator und dessen Herstellung, sowie die Verwendung des Katalysators in einem Verfahren zur Dehydratisierung von Alkoholen oder Polyalkoholen, insbesondere zur Herstellung von Furanverbindungen in wässriger Lösung. In diesem Zusammenhang stellt der Katalysator einen wesentlichen Bestandteil des erfindungsgemäßen Verfahrens zur Herstellung von Furfural oder 5-Hydroxymethylfurfural dar.

Der heterogene Katalysator umfasst BPO₄, AlPO₄, GaPO₄, Ti₃(PO₄)₄, FePO₄, und/oder Mischungen daraus.

Der heterogene Katalysator hat vorzugsweise eine spezifische Oberfläche kleiner 200 m²/g, insbesondere kleiner 160 m²/g. In einer besonderen Ausführungsform hat der heterogene Katalysator eine spezifische Oberfläche in einem Bereich von 100 bis 200 m²/g, insbesondere 130 bis 180 m²/g.

Die Katalysatoren eignen sich für die Dehydratisierung von Alkoholen und Polyalkoholen. Insbesondere eignen sich die Katalysatoren für eine Dehydratisierung von Aldosen und Ketosen, sowie deren Polymere und daraus ableitbare Produkte. In diesem Zusammenhang ist die Dehydratisierung von Sacchariden von besonderer Bedeutung. In einer bevorzugten Ausführungsform weist der Katalysator ein geringes Löslichkeitsprodukt in Wasser auf, welches das bevorzugte Reaktionsmedium darstellt. Bei phosphathaltigen Katalysatoren, wobei das Phosphat das Anion darstellt erfolgt aufgrund der geringen Löslichkeit der Abbau des katalytisch aktiven Materials bei Kontakt mit Wasser nur sehr langsam.

Es sind verschiedene Verfahren für die Herstellung eines heterogenen Katalysators denkbar. Zumeist erfolgt die Herstellung des Katalysators über entsprechende Vorläuferverbindungen oder deren Vorstufen.

Eine Ausführungsform des erfindungsgemäßen Verfahrens umfasst zumindest folgende Schritte:

### a) Bereitstellen einer Lösung aus einer Verbindung, welche das Metall, Halbmetall oder Übergangsmetall enthält.

Vorzugsweise werden Vorläuferverbindung oder ihre Vorstufen verwendet und in einem geeigneten Lösungsmittel verteilt, das vorzugsweise eine Säure, insbesondere Phosphorsäure enthält. In einer bevorzugten Ausführungsform wird die Metall-, Halbmetall- oder Übergangsmetallverbindung zerkleinert, so dass ein feines Pulver erhalten wird, welches anschließend in einem geeigneten Lösemittel verteilt wird. In einer besonderen Ausführungsform wird das feine Pulver molekulardispers verteilt, so dass eine homogene Lösung erhalten wird.

### b) Erhitzen der Lösung, welche das Metall, Halbmetall oder Übergangsmetall enthält.

Vorzugsweise wird die Lösung bis zum bis zum Siedepunkt des eingesetzten Lösemittels erhitzt. Vorzugsweise ist spätestens jetzt die Metall-, Halbmetall- oder Übergangsmetallverbindung vollständig gelöst.

### c) Ausfällen und isolieren des Katalysators

Durch Zugabe eines geeigneten Reagenz oder Abkühlen der Lösung wird der Katalysator ausgefällt.

In einer besonderen Ausführungsform wird der Katalysator in Gegenwart eines geeigneten Trägermaterials ausgefällt. Typische Trägermaterialien sind Ruß, Silicagel, Zeolithe oder Metalloxide. In einer weiteren besonderen Ausführungsform wird der Katalysator in einer geeigneten Säure, vorzugsweise H₂SO₄, getränkt und imprägniert, unter Imprägnieren ist in diesem Zusammenhang eine Oberflächenbehandlung des Katalysators zu verstehen. Bei einer Behandlung mit H₂SO₄ wird die Oberfläche des Katalysators sulfatiert.

Der Katalysator wird anschließend über bekannte Verfahren aus der Lösung isoliert, vorzugsweise über ein Filtrationsverfahren.

### d) Kalzinierung des Katalysators.

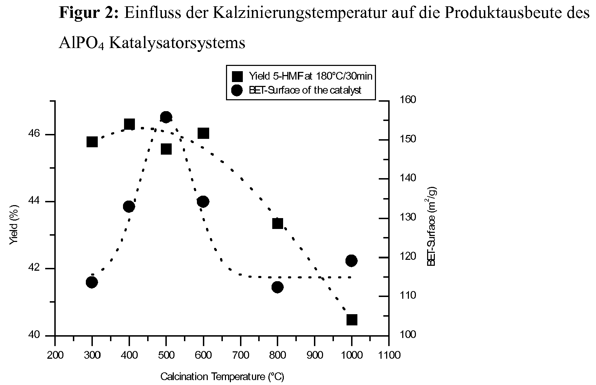

Der heterogene Katalysator wird anschließend kalziniert. Die Kalzinierung wird in einem Bereich von 200 bis 1200°C, vorzugsweise in einem Bereich von 200 bis 1000°C, besonders bevorzugt in einem Bereich von 400 bis 1000°C durchgeführt. In einer bevorzugten Ausführungsform wird die Kalzinierung in einem Bereich von 400 bis 600°C, insbesondere 450 bis 550°C durchgeführt. Durch die Kalzinierung werden flüssige Rückstände entfernt und der Katalysator aktiviert. Wie in Figur 2 dargestellt hat die Temperatur während der Kalzinierung einen entscheidenden Einfluss auf die Aktivität des Katalysators und dessen Oberfläche.

Optional wird der heterogene Katalysator getrocknet. Die Trocknung erfolgt in einem Temperaturbereich von 75°C bis 250°C, vorzugsweise von 100°C bis 200°C, insbesondere bei 105°C und über einen Zeitraum von 1 bis 48 Stunden, vorzugsweise 1 bis 24, insbesondere 12 bis 24. In einer bevorzugten Ausführungsform erfolgt die Trocknung zusätzlich zu der Klazinierung, insbesondere vor der Klazinierung.

In einer Ausführungsform sind die Vorläuferverbindungen Metalloxide und/oder Übergangsmetalloxide und/oder Halbmetalloxide. In einer bevorzugten Ausführungsform sind die Vorläuferverbindungen Metallchloride und/oder Übergangsmetallchloride und/oder Halbmetallchloride. In einer weiteren bevorzugten Ausführungsform der Erfindung sind die Vorläuferverbindungen Metallnitrate und/oder Übergangsmetallnitrate und/oder Halbmetallnitrate.

In einer Ausführungsform wird Aluminiumphosphat aus der Vorläuferverbindung Aluminiumnitrat hergestellt. Dabei wird das Aluminiumnitrat zunächst in Wasser gelöst und anschließend durch Zugabe von H₃PO₄ in Aluminiumphosphat überführt. Die Reaktionslösung wird daraufhin durch Zugabe einer geeigneten Base, vorzugsweise Ammoniak, neutralisiert und Aluminiumphosphat ausgefällt.

Al(NO₃)₃ + H₃PO₄ → AlPO₄ + 3 HNO₃

vorzugsweise

Al(NO₃)₃ (aq) + H₃PO4 → AlPO₄(aq) + 3 HNO₃

Die Vorstufen der Vorläuferverbindungen können Hydroxide sein. Die Metallhydroxide und/oder Übergangsmetallhydroxide und/oder Halbmetallhydroxide können in Gegenwart geeigneter Säuren, vorzugsweise geeigneter anorganischer Säuren, insbesondere HCl oder HNO₃, in die entsprechenden Chloride oder Nitrate überführt werden.

In einer Ausführungsform wird Aluminiumphosphat aus der Vorstufe Aluminiumhydroxid hergestellt. Dabei wird zunächst das Aluminiumhydroxid durch Zugabe von HNO₃ in Aluminiumnitrat überführt. Anschließend wird das Aluminiumnitrat durch Zugabe von H₃PO₄ in Aluminiumphosphat überführt. Die Reaktionslösung wird daraufhin durch Zugabe einer geeigneten Base, vorzugsweise Ammoniak, neutralisiert und das Aluminiumphosphat ausgefällt.

Al(OH)₃ + 3 HNO₃ → Al(NO₃)₃ + 3 H₂O

Al(NO₃)₃ + H₃PO₄ → AlPO₄ + 3 HNO₃

vorzugsweise

Al(OH)₃(s) + 3 HNO₃ → Al(NO₃)₃(aq) + 3 H₂O

Al(NO₃)₃(aq) + H₃PO₄ → AlPO₄ (aq)+ 3 HNO₃

Die Herstellung der Katalysatoren aus Metallchloriden und/oder Übergangsmetallchloriden und/oder Halbmetallchloriden erfolgt analog. Dabei wird zunächst das Metallhydroxid und/oder Übergangsmetallhydroxid und/oder Halbmetallhydroxid durch Zugabe von HCl in das entsprechende Chlorid überführt. Anschließend wird das Chlorid durch Zugabe von H₃PO₄ in das entsprechende Phosphat überführt. Die Reaktionslösung wird daraufhin durch Zugabe einer geeigneten Base, vorzugsweise NaOH oder KOH, neutralisiert und der Katalysator ausgefällt.

In einer weiteren bevorzugten Ausführungsform wird der Katalysator durch eine Verdünnung der Reaktionslösung, insbesondere mit Wasser, ausgefällt.

Aluminiumphosphat wird als heterogener Katalysator für das erfindungsgemäße Verfahren zur Herstellung von Furanverbindungen, insbesondere von Furfural und 5-Hydroxymethylfurfural besonders bevorzugt.

### BEISPIELE

(Nur Ausführungsbeispiele, welche unter die Ansprüche fallen sind Teil der Erfindung)

### Katalysatoren

Die Metalloxide und/oder Übergangsmetalloxide und/oder Halbmetalloxide sowie die Metallhydroxide und/oder Übergangsmetallhydroxide und/oder Halbmetallhydroxide sind kommerzielle Materialien und wurden zugekauft.

Die Metallphosphate und/oder Übergangsmetallphosphate und/oder Halbmetallphosphate wurden bis auf Galliumphosphat nach dem erfindungsgemäßen Verfahren aus den Nitraten (Al, Fe), Hydroxiden (Al), Oxiden (Al), Chloriden(Ti) oder Oxidchloriden (Zr), Säuren (B) hergestellt.

### Analytik

Die wässrigen Reaktionslösungen werden mittels einer quantitativen HPLC-Messung untersucht. Die Messung erfolgt mit einem Agilent HPLC 1100 mit RID 1200 Detektor. Dabei wird die HPLC mit H₂SO₄ (0,005 mol/L), mit einer Flussrate von 0.6 mL/min, bei 50°C beschickt. Die Säule ist eine Aminex 87H (300 x 7,8 mm), die Vorsäule eine Bio RAD 125-0129. Detektiert werden D-Fructose, D-Glucose, D-Xylose, 5-Hydroxymethylfurfural und Furfural. Außerdem können Ameisensäure, Essigsäure, Milchsäure und Lävulinsäure in unterschiedlichen Mengen, infolge einer Zersetzung der Produkte, auftreten.

### Batchbetrieb

Sämtliche Versuche zur Herstellung von Furanverbindungen nach einem Batchverfahren wurden in einem 100 mL Hochdruckstahlautoklav mit externem Heizelement durchgeführt (Autoclave Engineers, USA). Der Reaktor ist mit einem Magnetrührer und einem Gaseinlassventil ausgestattet. Über das Gaseinlassventil kann der Reaktor mit einem Inertgas geflutet werden, um Folgereaktionen mit Luftsauerstoff bei den hohen Temperaturen zu vermeiden.

### Kontinuierlicher Betrieb

Sämtliche Versuche zur Herstellung von Furanverbindungen nach einem kontinuierlichen Verfahren wurden in einem 100 mL Hochdruckstahlautoklav mit externer Beheizung und zumindest einer HPLC-Pumpe (JASCO, Germany) durchgeführt. Die Syntheseanlage ist in Figur 1 dargestellt. Das Vermischen von Wasser und Saccharidlösung erfolgt erst unmittelbar vor dem Reaktorgefäß und erlaubt so eine hervorragende Kontrolle über die Temperatur und damit über die Dauer der Reaktion. Ein Sintermetallfilter am Ausgang des Hochdruckstahlautoklav verhindert dabei das Austreten des Katalysators, der durch einen Magnetrührfisch im Reaktorvolumen dispergiert wird.

### Synthese von Aluminiumphosphat

Die Synthese von Aluminiumphosphat erfolgt über die Vorläuferverbindung Aluminiumnitrat. Das Aluminiumnitrat wird zunächst in Wasser gelöst, mit einem Überschuss an konzentrierter Phosphorsäure vermengt und für 8 Stunden bei 100°C unter Rückfluss erhitzt. Daraufhin wird das gebildete Aluminiumphosphat durch Zugabe von Ammoniak gefällt und abfiltriert. Das Filtrat wird mit Wasser gewaschen, bei 105°C für 24 Stunden getrocknet und anschließend für 4 Stunden bei 400°C kalziniert.

Die Synthese von geträgertem Aluminiumphosphat erfolgt ebenfalls über die Vorläuferverbindung Aluminiumnitrat. Das Trägermaterial, insbesondere α-Al₂O₃, wird mit einer wässrigen Aluminiumnitratlösung vermengt. Nach 60 Minuten wird ein Überschuss an konzentrierter Phosphorsäure zugegeben und für 8 Stunden bei 100°C unter Rückfluss erhitzt. Anschließend wird das Aluminiumphosphat auf das mit Aluminiumphosphat getränkte Trägermaterial durch überstöchiometrische Zugabe von Ammoniak gefällt, mit ausreichend Wasser gewaschen bei 105°C für 24 Stunden getrocknet und anschließend für 4 Stunden bei 400°C kalziniert

### Herstellung von Furfural und 5-Hydroxymethylfurfural

Das erfindungsgemäße Verfahren zur Herstellung von Furanverbindungen wird für die Herstellung von Furfural und 5-Hydroxymethylfurfural aus Xylose und Fructose, in Gegenwart verschiedener heterogener Katalysatoren untersucht.

### Vergleichsbeispiel 1

Herstellung von Furfural aus Xylose ohne Katalysator im Batchbetrieb 10 g Xylose werden mit 190 g vollentsalztem Wasser unter Rühren vereinigt, so dass eine Lösung mit 5 % Xylose erhalten wird. 50 mL der Lösung werden in einen 100 mL Reaktor mit externer Beheizung gegeben. Anschließend wird der Reaktor verschlossen und für 5 Minuten mit einer Stickstoffatmosphäre inertisiert. Der Reaktor wird auf einen Vordruck von 5 bar und einer Temperatur von 160°C eingestellt. Anschließend wird der Reaktor erhitzt. Sobald in dem Reaktor eine Temperatur von 160°C erreicht ist wird das Reaktionsgemisch bei 305 rpm gerührt. Nach einer Reaktionszeit von 60 Minuten wird die Heizvorrichtung entfernt und der Reaktor an Luft abgekühlt, bis die Temperatur im Inneren des Reaktors 40°C beträgt. Anschließend wird der im Inneren des Reaktors anliegende Überdruck durch einen am Reaktor angebrachten Gasauslass entspannt und der Reaktor geöffnet. Die Reaktionslösung wird ausgewogen und mittels HPLC quantitativ auf ihre Inhaltsstoffe untersucht.

### Vergleichsbeispiel 2

Die Verfahrensführung erfolgt wie unter Beispiel 1 beschrieben, allerdings werden nicht 500 mg pulverförmiges Aluminiumphosphat (AlPO₄) in den Reaktor gegeben sondern 2,5 mL 98 %ige H₂SO₄, was einem Verhältnis von Xylose zu H₂SO₄ von 1:1,8 entspricht.

### Beispiel 1

Herstellung von Furfural aus Xylose mit AlPO₄ im Batchbetrieb 25 g Xylose werden mit 475 g vollentsalztem Wasser unter Rühren vereinigt, so dass eine Lösung mit 5 % Xylose erhalten wird. 50 mL der Lösung werden in einen 100 mL Reaktor mit externer Beheizung gegeben. Anschließend werden 500 mg pulverförmiges Aluminiumphosphat (AlPO₄) in den Reaktor gegeben. Daraufhin wird der Reaktor verschlossen und für 5 Minuten mit einer Stickstoffatmosphäre beaufschlagt. Der Reaktor wird auf einen Vordruck von 5 bar Stickstoff und eine Temperatur von 160°C eingestellt. Anschließend wird der Reaktor erhitzt. Sobald in dem Reaktor eine Temperatur von 160°C erreicht ist wird das Reaktionsgemisch bei 305 rpm gerührt. Nach einer Reaktionszeit von 60 Minuten wird die Heizvorrichtung entfernt und der Reaktor an Luft abgekühlt, bis die Temperatur im Inneren des Reaktors 40°C beträgt. Anschließend wird der im Inneren des Reaktors anliegende Überdruck durch einen am Reaktor angebrachten Gasauslass entspannt und der Reaktor geöffnet. Die Reaktionslösung wird ausgewogen und das Aluminiumphosphat mit einem Faltenfilter (Whatman Grade 589) abgetrennt. Das Aluminiumphosphat wird für 24 Stunden bei 105°C getrocknet und ebenfalls ausgewogen. Die Reaktionslösung wird mittels HPLC quantitativ auf ihre Inhaltsstoffe untersucht.

### Beispiele 2 bis 6

Die Verfahrensführung erfolgt wie unter Beispiel 1 beschrieben, allerdings werden nicht 500 mg pulverförmiges Aluminiumphosphat (AlPO4) in den Reaktor gegeben sondern:

| | |
|---|---|
| 500 mg Alumosilikat (5 % Al₂O₃) | (Beispiel 2) |
| 500 mg Alumosilikat (30 % Al₂O₃) | (Beispiel 3) |
| 500 mg Alumosilikat (40 % Al₂O₃) | (Beispiel 4) |
| 500 mg Al₂O₃ | (Beispiel 5) |
| 500 mg TiO₂ (I) | (Beispiel 6) |

### Beispiele 7 bis 8

Die Verfahrensführung erfolgt wie unter Beispiel 1 beschrieben, mit Ausnahme der Reaktionstemperatur, welche in Beispiel 7 auf 180°C und in Beispiel 8 auf 200°C erhöht wird. Außerdem wird die Reaktionszeit auf 30 min verkürzt.

Tabelle 1 fasst die im Batchbetrieb von verschiedenen heterogenen Katalysatoren erzielten Ergebnisse bei der Herstellung von Furfural aus einer wässrigen Xyloselösung zusammen. Als Vergleichsbeispiele dienen zum einen die unkatalysierte Reaktion (Vergleich 1) und zum anderen die homogen katalysierte Reaktion mit H₂SO₄ (Vergleich 2).

**Tabelle 1: Herstellung von Furfural aus Xylose**

| | Katalysator | Temperatur | Reaktionszeit | Umsatz Xylose | Ausbeute Furfural |
|---|---|---|---|---|---|
| | | (°C) | (min) | (%) | (%) |
| Vergleich 1 | Ohne | 160 | 60 | 7,91 | 6,24 |
| Vergleich 2 | H₂SO₄ | 160 | 60 | 70,52 | 34,54 |
| Beispiel 1 | AlPO₄ | 160 | 60 | 17,50 | 10,48 |
| Beispiel 2 | Alumosilikat (5 % Al₂O₃) | 160 | 60 | 84,64 | 16,75 |
| Beispiel 3 | Alumosilikat (30 % Al₂O₃) | 160 | 60 | 78,42 | 18,94 |
| Beispiel 4 | Alumosilikat (40 % Al₂O₃) | 160 | 60 | 54,52 | 15,49 |
| Beispiel 5 | Al₂O₃ | 160 | 60 | 83,99 | 18,28 |
| Beispiel 6 | TiO₂ (I) | 160 | 60 | 79,94 | 32,57 |
| Beispiel 7 | AlPO₄ | 180 | 30 | 27,13 | 18,08 |
| Beispiel 8 | AlPO₄ | 200 | 30 | 89,02 | 46,44 |

### Herstellung von 5-Hydroxymethylfurfural aus Fructose ohne Katalysator im Batchbetrieb

### Vergleichsbeispiel 3

25 g Fructose werden mit 475 g vollentsalztem Wasser unter Rühren vereinigt, so dass eine Lösung mit 5 % Fructose erhalten wird. 50 mL der Lösung werden in einen 100 mL Reaktor mit externer Beheizung gegeben. Anschließend wird der Reaktor verschlossen und für 5 Minuten mit einer Stickstoffatmosphäre beaufschlagt. Der Reaktor wird auf eine Temperatur von 180°C eingestellt. Anschließend wird der Reaktor erhitzt. Sobald in dem Reaktor eine Temperatur von 180°C erreicht ist wird das Reaktionsgemisch bei 305 rpm gerührt. Nach einer Reaktionszeit von 30 Minuten wird die Heizvorrichtung entfernt und der Reaktor an Luft abgekühlt, bis die Temperatur im Inneren des Reaktors 40°C beträgt. Anschließend wird der im Inneren des Reaktors anliegende Überdruck durch einen am Reaktor angebrachten Gasauslass entspannt und der Reaktor geöffnet. Die Reaktionslösung wird ausgewogen und mittels HPLC quantitativ auf ihre Inhaltsstoffe untersucht.

### Vergleichsbeispiel 4

25 g Fructose werden mit 475 g vollentsalztem Wasser unter Rühren vereinigt, so dass eine Lösung mit 5 % Fructose erhalten wird. 50 mL der Lösung werden in einen 100 mL Reaktor mit externer Beheizung gegeben. Anschließend werden 2,5 mL 98 %ige H₂SO₄ in den Reaktor gegeben, was einem Verhältnis von Fructose zu H₂SO₄ von 1:1,8 entspricht. Daraufhin wird der Reaktor verschlossen und für 5 Minuten mit einer Stickstoffatmosphäre beaufschlagt. Der Reaktor wird auf eine Temperatur von 180°C eingestellt. Anschließend wird der Reaktor erhitzt. Sobald in dem Reaktor eine Temperatur von 180°C erreicht ist wird das Reaktionsgemisch bei 305 rpm gerührt. Nach einer Reaktionszeit von 10 Minuten wird die Heizvorrichtung entfernt und der Reaktor an Luft abgekühlt, bis die Temperatur im inneren des Reaktors 40°C beträgt. Anschließend wird der im inneren des Reaktors anliegende Überdruck durch einen am Reaktor angebrachten Gasauslass entspannt und der Reaktor geöffnet. Die Reaktionslösung wird ausgewogen. Die Reaktionslösung wird mittels HPLC quantitativ auf ihre Inhaltsstoffe untersucht.

### Herstellung von 5-Hydroxymethylfurfural aus Fructose mit Katalysator im Batchbetrieb

### Beispiel 9

25 g Fructose werden mit 475 g vollentsalztem Wasser unter Rühren vereinigt, so dass eine Lösung mit 5 % Fructose erhalten wird. 50 mL der Lösung werden in einen 100 mL Reaktor mit externer Beheizung gegeben. Anschließend werden 500 mg pulverförmiges Aluminiumphosphat (AlPO₄) in den Reaktor gegeben. Daraufhin wird der Reaktor verschlossen und für 5 Minuten mit einer Stickstoffatmosphäre beaufschlagt. Der Reaktor wird auf eine Temperatur von 180°C eingestellt.

Anschließend wird der Reaktor erhitzt. Sobald in dem Reaktor eine Temperatur von 180°C erreicht ist wird das Reaktionsgemisch bei 305 rpm gerührt. Nach einer Reaktionszeit von 30 Minuten wird die Heizvorrichtung entfernt und der Reaktor an Luft abgekühlt, bis die Temperatur im Inneren des Reaktors 40°C beträgt. Anschließend wird der im Inneren des Reaktors anliegende Überdruck durch einen am Reaktor angebrachten Gasauslass entspannt und der Reaktor geöffnet. Die Reaktionslösung wird ausgewogen und das Aluminiumphosphat mit einem Faltenfilter (Whatman Grade 589) abgetrennt. Das Aluminiumphosphat wird für 24 Stunden bei 105°C getrocknet und ebenfalls ausgewogen. Die Reaktionslösung wird mittels HPLC quantitativ auf ihre Inhaltsstoffe untersucht.

### Beispiele 10 bis 17

Die Verfahrensführung erfolgt wie unter Beispiel 9 beschrieben, allerdings werden nicht 500 mg pulverförmiges Aluminiumphosphat (AlPO4) in den Reaktor gegeben sondern:

| | |
|---|---|
| 500 mg TiO₂ (I) | (Beispiel 10) |
| 500 mg SiAl (40 %) | (Beispiel 11) |
| 500 mg FePO₄ | (Beispiel 12) |
| 500 mg Zr₃(PO₄)₄ | (Beispiel 13) |
| 500 mg ZrO₂ | (Beispiel 14) |
| 500 mg TiO₂ (II) | (Beispiel 15) |
| 500 mg GaPO₄ | (Beispiel 16) |
| 500 mg BPO₄ | (Beispiel 17) |

### Beispiel 18

Die Verfahrensführung erfolgt wie unter Beispiel 9 beschrieben, mit Ausnahme der Reaktionstemperatur welche auf 200°C erhöht und die Reaktionszeit auf 10 min verkürzt wird.

Tabelle 2 fasst die im Batchbetrieb von verschiedenen heterogenen Katalysatoren erzielten Ergebnisse, bei der Herstellung von 5-Hydroxymethylfurfural aus einer wässrigen Fructoselösung, zusammen. Als Vergleichsbeispiele dienen zum einen die unkatalysierte Reaktion (Vergleich 1) und zum anderen die homogen katalysierte Reaktion mit H₂SO₄ (Vergleich 2).

**Tabelle 2: Herstellung von 5-Hydroxymethylfurfural**

| | Katalysator | Temperatur | Verweilzeit | Umsatz Fructose | Ausbeute HMF |
|---|---|---|---|---|---|
| | | [°C] | [min] | [%] | [%] |
| Vergleich 3 | Ohne | 180 | 30 | 69,69 | 39,50 |
| Vergleich 4 | H₂SO₄ | 160 | 10 | 99,49 | 5,69 |
| Beispiel 9 | AlPO₄ (400°C) | 180 | 30 | 81,63 | 46,31 |
| Beispiel 10 | TiO₂ (I) | 180 | 30 | 94,47 | 22,44 |
| Beispiel 11 | Alumosilikat (40% Al₂O₃) | 180 | 30 | 79,31 | 23,93 |
| Beispiel 12 | FePO₄ | 180 | 30 | 94,00 | 41,61 |
| Beispiel 13 | Zr₃(PO₄)₄ | 180 | 30 | 86,81 | 40,40 |
| Beispiel 14 | ZrO₂ | 180 | 30 | 74,48 | 45,46 |
| Beispiel 15 | TiO₂ (II) | 180 | 30 | 79,23 | 46,36 |
| Beispiel 16 | GaPO₄ | 180 | 30 | 75,11 | 43,48 |
| Beispiel 17 | BPO₄ | 180 | 30 | 71,21 | 44,49 |
| Beispiel 18 | AlPO₄ (400°C) | 200 | 10 | 95,33 | 44,35 |

Bei der Herstellung von Furfural und 5-Hydroxymethylfurfural im Batchbetrieb nach dem erfindungsgemäßen Verfahren können hohe Umsätze erzielt werden, wobei mit dem homogen katalysierten Verfahren vergleichbare Ausbeuten erzielt werden, insbesondere bei einer Reaktionstemperatur von 160°C bis 180°C und einer Reaktionszeit von 10 bis 60 Minuten. Die bei dem homogen katalysierten Verfahren erzielten Umsatzraten und Ausbeuten können mit dem erfindungsgemäßen Verfahren bei gleichen Reaktionsbedingungen erzielt werden, insbesondere mit TiO₂. Bei einer Reaktionstemperatur von 200°C und einer Reaktionszeit von 60 Minuten können Ausbeute und Umsatzrate des homogen katalysierten Verfahrens sogar übertroffen werden, wenn AlPO₄ als heterogener Katalysator zur Herstellung von Furfural aus Xylose eingesetzt wird.

Weiterhin ergibt sich, dass bei der Herstellung von 5-Hydroxymethylfurfural nach dem erfindungsgemäßen Verfahren ebenfalls hohe Umsätze erzielt werden, welche deutlich über den im homogen katalysierten Verfahren liegen. Die dabei erzielten Ausbeuten liegen ebenfalls deutlich über den im homogen katalysierten Verfahren, insbesondere bei einer Reaktionstemperatur von 180°C und einer Reaktionszeit von 30 Minuten. Das homogen katalysierte Verfahren zeigt bei den milden Reaktionsbedingungen nur eine äußerst geringe Umsatzrate, während mit dem erfindungsgemäßen Verfahren hohe Umsatzraten und Ausbeuten erzielt werden, insbesondere mit AlPO₄ (400°C), wobei AlPO₄ (400°C) einen Aluminiumphosphatkatalysator bezeichnet, der bei 400°C kalziniert wurde.

Die Reaktionstemperatur und die Reaktionszeit haben einen großen Einfluss auf die bei der Dehydratisierung erzielten Ausbeuten. Dabei werden in den Bereichen von 160°C bis 200°C und 10 bis 60 Minuten stark voneinander abweichende Ergebnisse erzielt.

Typischerweise werden hohe Ausbeuten bei kurzen Reaktionszeiten und hohen Temperaturen erwartet. Die Reaktionsparameter, insbesondere die Reaktionstemperatur und Reaktionszeit, sowie eingesetzten Edukte und können ohne weiteres auf eine kontinuierlich betriebene Syntheseanlage übertragen werden.

### Herstellung von 5-Hydroxymethylfurfural aus Fructose mit Katalysator im kontinuierlichen Betrieb

Die Herstellung von 5-Hydroxymethylfurfural aus Fructose im kontinuierlichen Betrieb erfolgt mit einer Syntheseanlage mit Rührkesselreaktor gemäß Figur 1. 140 g Fructose werden mit 860 g vollentsalztem Wasser unter Rühren vereinigt, so dass eine Lösung mit 14% Fructose erhalten wird, welche über einen der beiden Stränge der Syntheseanlage durch die Anlage zur Mischstelle und anschließend in den Reaktor gefördert wird. 1000 mg des pulverförmigen Aluminiumphosphats (AlPO₄) werden in einen 100 mL Reaktor (1) mit externer Beheizung und einem freien Reaktorvolumen von 70 mL gegeben. Die Kühlung des Reaktoraustrags erfolgt mit einem Kühlsystem (17) das eine Temperatur ≤ 20°C hat. Das Aluminiumphosphat wird mit einem Magnetrührer bei 400 rpm in dem Reaktorvolumen dispergiert. Der Reaktor (1) wird über das Druckregelventil (15) auf einen Vordruck von 50 bar und über die Heizvorrichtung (16) auf eine Temperatur von 190°C eingestellt. Zum Anfahren und Fluten der Anlage werden 4,2 mL/min und 8,14 mL/min vollentsalztes Wasser über die beiden Hochdruckpumpen (11) und (12) durch die Anlage und in den Reaktor (1) gespeist. Der Vorwärmer (13) wird auf 230°C und der Vorwärmer (14) auf 190°C eingestellt. Die vor dem Reaktoreingang angeordnete Heizvorrichtung (16) wird auf 165°C und die externe Reaktorbeheizung auf 190°C eingestellt. Nach 50 Minuten ist in dem Reaktor eine konstante Temperatur von 190°C erreicht. Anschließend wird die Hochdruckpumpe (11) auf die Fructoselösung umgestellt. Die Hochdruckpumpe (11) fördert dabei 1,21 mL/min Fructoselösung und die Hochdruckpumpe (12) 2,34 mL/min vollentsalztes Wasser. Am Reaktoreingang (56) liegt damit eine Fructoselösung mit einer Fructosekonzentration von etwa 5 % an. Die mittlere Verweilzeit der Fructoselösung in dem Reaktor (1) beträgt 17,5 Minuten. Nach 81,09 Minuten erreicht die Reaktionslösung mit dem gebildeten 5-Hydroxymethylfurfural den Anlagenausgang. Es kann nun kontinuierlich die gebildete Produktlösung am Anlagenausgang entnommen werden. Zum Abfahren der Anlage wird die Hochdruckpumpe (11) auf vollentsalztes Wasser umgestellt, sämtliche Vorwärmer ausgeschaltet und die Anlage für 90 min gespült und abgekühlt. Die erhaltene Produktlösung wird ausgewogen und die Reaktionslösung mittels HPLC quantitativ auf ihre Inhaltsstoffe untersucht.

Aus weiteren Vergleichsversuchen mit einem kontinuierlich betriebenen Reaktor zeigt sich, dass die Reaktionstemperatur und die Reaktionszeit einen großen Einfluss auf die von den verschiedenen erfindungsgemäßen Katalysatorsystemen bei der Dehydratisierung von Fructose erzielten Ausbeuten haben. Dabei kommt es in den Bereichen von 180°C bis 200°C und 5 bis 30 Minuten zu stark voneinander abweichenden Ergebnissen bei den verschiedenen Katalysatorsystemen.
Die Reaktionsparameter, insbesondere die Reaktionstemperatur und Reaktionszeit, sowie die eingesetzten Edukte und Eduktlösungen können ohne weiteres auf eine Syntheseanlage, welche nicht kontinuierlich betrieben wird, übertragen werden.

### Kalzinierung

Vergleichsversuche zur Optimierung des erfindungsgemäßen Verfahrens zur Herstellung eines heterogenen Katalysators zeigen, dass insbesondere die Kalzinierung von besonderer Bedeutung für die Aktivität des daraus erhaltenen Katalysators ist. Figur 2 zeigt den Einfluss der Kalzinierungstemperatur auf die spezifische Oberfläche des Katalysators und die damit bei gleichen Reaktionsbedingungen erzielten Ausbeuten. Dabei wurden über die mittels BET bestimmten N₂-Adsorptionsisothermen die spezifische Oberflächen der bei verschiedenen Temperaturen kalzinierten AlPO₄-Katalysatoren bestimmt. Die optimale Kalzinierungstemperatur liegt demzufolge zwischen 400 und 600°C, insbesondere zwischen 450 und 550°C, bezogen auf die Ausbeute an 5-Hydroxymethylfurfural. Die spezifische Oberfläche des Katalysators ist dabei zwischen 130 und 160 m²/g.

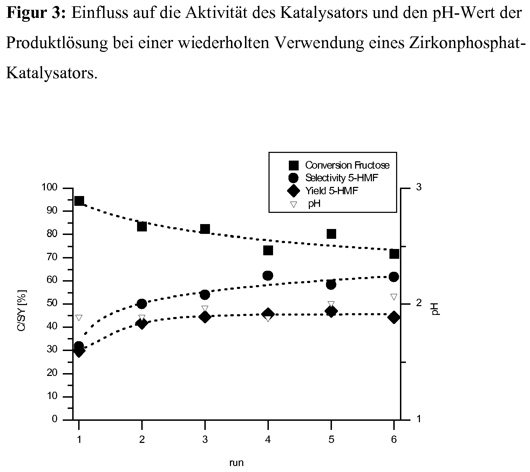

Figur 3 zeigt, dass die katalytische Aktivität des Zr₃(PO₄)₄ Katalysators auch bei wiederholten Durchläufen erhalten bleibt, was auf eine hohe Lebensdauer schließen lässt. Dies ist insbesondere für industrielle Anwendungen interessant.

Das erfindungsgemäße Herstellungsverfahren ist geeignet, um Furfural und 5-Hydroxymethylfurfural unter milden Reaktionsbedingungen, aus nachhaltigen Rohstoffen, herzustellen. Mit dem erfindungsgemäßen Herstellungsverfahren können dabei die Umsätze und Ausbeuten aus dem homogen katalysierten Verfahren übertroffen werden, bei einer gleichzeitig verbesserten Prozessführung und verringerten Produktionskosten. Ein weiterer Vorteil des erfindungsgemäßen

Verfahrens gegenüber dem homogen katalysierten Verfahren ist die stark vereinfachte Rückgewinnung des Katalysators, insbesondere bei einem kontinuierlich betriebenen Reaktor. Die Vergleichsversuche zeigen den starken Einfluss der Reaktionsbedingungen auf die erreichbaren Umsätze und Ausbeuten sowohl bei der Herstellung des Katalysators als auch bei der Herstellung der Furanverbindungen.

## Patentansprüche

1. Verfahren zur Herstellung von Furfural oder 5-Hydroxymethylfurfural, unter Verwendung eines heterogenen Katalysators in wässriger Lösung, **dadurch gekennzeichnet, dass** der heterogene Katalysator FePO₄, GaPO₄, BPO₄, Ti₃(PO₄)₄, AlPO₄ und/oder Mischungen davon enthält, wobei das Verfahren das Bereitstellen von mindestens einem Saccharid in Form einer wässrigen Lösung umfasst.

2. Verfahren gemäß Anspruch 1, wobei das mindestens eine Saccharid Xylose und/oder Fructose ist.

3. Verfahren nach Anspruch 1 oder 2, worin das Saccharid dehydratisiert wird.

4. Verfahren gemäß einem der vorgehenden Ansprüche umfassend die Schritte
a) bereitstellen einer Saccharidlösung,
b) zugeben eines heterogenen Katalysators,
c) erhitzen,
d) abtrennen des heterogenen Katalysators optional mittels Filtration.

5. Verfahren gemäß Anspruch 4, **dadurch gekennzeichnet, dass** die Saccharidlösung zumindest ein Saccharid umfasst, welches aus einer biogenen Quelle stammt oder über ein Bioraffinationsverfahren gewonnen wurde und in wässriger Lösung eingesetzt wird.

6. Verfahren gemäß einem der vorgehenden Ansprüche **dadurch gekennzeichnet, dass** es im Batchbetrieb oder im kontinuierlichen Betrieb durchgeführt werden kann.

7. Verfahren gemäß einem der vorgehenden Ansprüche dadurch charakterisiert, dass der heterogene Katalysator eine spezifische Oberfläche in einem Bereich von 100 bis 200 m²/g aufweist.

8. Verfahren gemäß einem der vorhergehenden Ansprüche, wobei ein heterogerner Katalysator eingesetzt wird dessen Herstellung einen Kalzinierungsschritt bei 400°C bis 1000°C umfasst.

9. Verfahren gemäß einem der vorgehenden Ansprüche dadurch charakterisiert, dass der heterogene Katalysator ungeträgert oder geträgert ist.

10. Verwendung eines heterogenen Katalysators umfassend FePO₄, GaPO₄, BPO₄, Ti₃(PO₄)₄, AlPO₄ und/oder Mischungen davon zur Dehydratisierung von Sacchariden, wobei das Saccharid in Form einer wässrigen Lösung vorliegt, und wobei Furfural oder 5-Hydroxymethylfurfural erhalten werden.

## Claims

1. Process for producing furfural or 5-hydroxy-methylfurfural using a heterogeneous catalyst in aqueous solution, **characterized in that** the heterogeneous catalyst comprises FePO₄, GaPO₄, BPO₄, Ti₃(PO₄)₄, AlPO₄ and/or mixtures thereof, wherein the process comprises providing at least one saccharide in the form of an aqueous solution.

2. Process according to Claim 1, wherein the at least one saccharide is xylose and/or fructose.

3. Process according to Claim 1 or 2, wherein the saccharide is dehydrated.

4. Process according to any of the preceding claims comprising the steps of
(a) providing a saccharide solution,
(b) adding a heterogeneous catalyst,
(c) heating,
(d) removing the heterogeneous catalyst optionally by filtration.

5. Process according to Claim 4, **characterized in that** the saccharide solution comprises at least one saccharide which originates from a biogenic source or has been obtained by a biorefining process and is employed in aqueous solution.

6. Process according to any of the preceding claims, **characterized in that** said process may be carried out in batch operation or in continuous operation.

7. Process according to any of the preceding claims, **characterized in that** the heterogeneous catalyst has a specific surface area in the range from 100 to 200 m²/g.

8. Process according to any of the preceding claims, wherein said process employs a heterogeneous catalyst whose production comprises a calcining step at 400°C to 1000°C.

9. Process according to any of the preceding claims, **characterized in that** the heterogeneous catalyst is unsupported or supported.

10. Use of a heterogeneous catalyst comprising FePO₄, GaPO₄, BPO₄, Ti₃(PO₄)₄, AlPO₄ and/or mixtures thereof for dehydrating saccharides, wherein the saccharide is in the form of an aqueous solution and wherein furfural or 5-hydroxymethylfurfural are obtained.

## Revendications

1. Procédé de fabrication de furfural ou de 5-hydroxyméthylfurfural, utilisant un catalyseur hétérogène en solution aqueuse, **caractérisé en ce que** le catalyseur hétérogène contient FePO₄, GaPO₄, BPO₄, Ti₃(PO₄)₄, AlPO₄ et/ou leurs mélanges, le procédé comprenant la préparation d'au moins un saccharide sous la forme d'une solution aqueuse.

2. Procédé selon la revendication 1, dans lequel ledit au moins un saccharide est le xylose et/ou le fructose.

3. Procédé selon la revendication 1 ou 2, dans lequel le saccharide est déshydraté.

4. Procédé selon l'une quelconque des revendications précédentes, comprenant les étapes suivantes :
a) la préparation d'une solution de saccharide,
b) l'ajout d'un catalyseur hétérogène,
c) le chauffage,
d) la séparation du catalyseur hétérogène éventuellement par filtration.

5. Procédé selon la revendication 4, **caractérisé en ce que** la solution de saccharide comprend au moins un saccharide qui provient d'une source biogène ou qui a été obtenu par un procédé de bioraffinage et est utilisé en solution aqueuse.

6. Procédé selon l'une quelconque des revendications précédentes, **caractérisé en ce qu'**il peut être réalisé en mode discontinu ou en mode continu.

7. Procédé selon l'une quelconque des revendications précédentes, **caractérisé en ce que** le catalyseur hétérogène présente une surface spécifique dans une plage allant de 100 à 200 m²/g.

8. Procédé selon l'une quelconque des revendications précédentes, dans lequel un catalyseur hétérogène dont la fabrication comprend une étape de calcination à une température de 400 °C à 1 000 °C est utilisé.

9. Procédé selon l'une quelconque des revendications précédentes, **caractérisé en ce que** le catalyseur hétérogène est non supporté ou supporté.

10. Utilisation d'un catalyseur hétérogène comprenant FePO₄, GaPO₄, BPO₄, Ti₃(PO₄)₄, AlPO₄ et/ou leurs mélanges pour la déshydratation de saccharides, le saccharide se présentant sous la forme d'une solution aqueuse et du furfural ou du 5-hydroxyméthylfurfural étant obtenu.
